# EUROPEAN PATENT APPLICATION

(11) **EP 4 813 933 A1**
(43) Date of publication of application: **30.09.2026**
(21) Application number: 25167059.2
(22) Date of filing: 28.03.2025
(51) Int. Cl.: C02F 3/34, C02F 101/34, C02F 103/36

(54) **A MIXTURE OF MICROORGANISMS THAT DECOMPOSES PHENOL COMPOUNDS FOUND IN REFINERY WASTEWATER**

(71) Applicant: Star Rafineri Anonim Sirketi, 34396 Istanbul (TR)
(72) Inventor: ALPAY UYANIKER, Tayyibe, 35800 Izmir (TR); PASHAYEVA, Bike, 35800 Izmir (TR); SARICAYIR, Selin, 35040 Izmir (TR); OZDEMIR, Guven, 35040 Izmir (TR); EZDESIR, Ayhan, 35800 Izmir (TR)
(74) Representative: Sevinç, Cenk

(57) **Abstract**

The invention relates to a bacterial culture mixture and the preparation method thereof to provide biodegradation of phenol compounds such as o-cresol, p-cresol, m-cresol, 2,3 dichlorophenol, 2,4 dichlorophenol, 3,4 dichlorophenol, 2,4 dinitrophenol, 2,5 dichlorophenol found in refinery wastewater. The bacterial mixture, which is the subject of the invention, comprises pure bacterial isolates isolated from wastewater system activated sludge belonging to the genera *Pseudomonas, Acinetobacter, Lysinibacillus, Bacillus, Kurthia, Comamonas, Microbacterium, Brevundimonas.*

## Description

### Technical Field of the Invention

The invention relates to a bacterial culture mixture and the preparation method thereof to provide biodegradation of phenol compounds such as o-cresol, p-cresol, m-cresol, 2,3 dichlorophenol, 2,4 dichlorophenol, 3,4 dichlorophenol, 2,4 dinitrophenol, 2,5 dichlorophenol found in refinery wastewater. Said bacterial mixture provides treatment of refinery wastewater that exhibits high toxic activity for the ecosystem due to the presence of phenolic compounds.

### State of the Art

Wastewater is defined as the water-borne waste material of a community, including all normal wastes originating from human activities and residences, as well as many forms of waste substances and compounds from industries and commercial establishments [1], and contains significant concentrations of solids, dissolved and particulate matter, microorganisms, nutrients, heavy metals and micropollutants [2]. The most severe impact of wastewater on the environment due to the components it contains is that it causes pollution and destruction of natural habitats and wildlife living in these habitats by exposing them to harmful chemicals that would not otherwise be present in the natural course of events. In addition, the composition of wastewater may include heavy metals, pathogens, salts, toxic chemicals, oil and grease, solids, nutrients, sludge, acids and bases, toxic organic compounds, organic and inorganic materials, thus posing numerous hazards for humans, animals and the environment. The wastewater treatment process applied to eliminate these hazards is the methods aimed at restoring the water used and/or polluted by humans or nature to a desired quality.

Although wastewater can exist in nature due to many sources and reasons, the factor that causes the most wastewater formation is in the industrial area and is released from refineries. The units owned by refineries produce wastewater containing high amounts of nitrogen, ammonia, methane, carbon dioxide, nitrogen oxides, hydrogen sulphide, mercaptans, chlorine and phenol, depending on the processed product, and the presence of these waters in nature without purification [3] poses a high danger to the ecosystem and the life of living beings it comprises. In particular, the direct or indirect discharge of wastewater from industrial activities into water bodies increases the amount of pollution of water with phenolic compounds to the maximum level. Phenol, which is highly soluble in water, is acceptable up to a concentration of 100 µg/L if the water is not to be chlorinated [4]. However, due to the by-products that may form as a result of chlorination, the total phenol content of water is expected to be below 1 µg/L [5]. According to EU standards, phenol is listed among the "substances that are undesirable to be present in high amounts" and the highest permissible concentration is determined as 0.5 µg/L C₆H₅OH (excluding phenols that do not react with chlorine). After chlorination of phenolic waters, the main reaction products are 2 and 4 chlorophenols, 2, 4 dichlorophenols and 2, 4, 6, trichlorophenols, and these products can generally cause taste and odour formation in water even at very low concentrations, in addition, they have toxic effects on humans, animals and aquatic life, can remain in the environment and biological systems for a long time due to their stability and bioaccumulation, and their interaction even at low concentrations damages red blood cells and the liver [6]. They can also form carcinogenic chlorophenols in the presence of chlorine. For this reason, phenolic compounds are considered priority water pollutants by the EPA (Environmental Protection Agency) and NPRI (National Pollutant Release Inventory) in the USA and Canada [7] and there are many methods in the state of the art for the treatment of these compounds from wastewater. These methods include distillation, air stripping, biological adsorption, chemical oxidation (chlorine dioxide, hydrogen peroxide, ozone), deep well injection, irradiation, extraction, electrochemical oxidation and chemical oxidation [8], but physicochemical cleaning or processing technologies have disadvantages due to their tendency to form secondary toxic intermediates, and methods such as extraction, adsorption and oxidation require high costs, can pollute groundwater, and are not effective in completely eliminating phenol compounds. Therefore, traditionally most widely used method has been the use of effective microorganisms capable of degrading toxic chemicals [9]. Bacteria, fungi and algae degrade phenol directly by using it as a sole carbon source or indirectly in the presence of another metabolic substrate, a process called co-metabolism. In particular, pure and mixed cultures of the genus *Pseudomonas* are the most widely used biomass species for the biodegradation of phenols and are believed to have good potential for different biotechnological applications. Specifically, *Pseudomonas putida* is widely used in the state of the art for the biodegradation of phenol due to its high removal efficiency [10]. However, treatment plants cannot show sufficient efficiency in the treatment of wastewater due to reasons such as physicochemical conditions (oxygen status, presence of toxic substances, pH, temperature, etc.), microorganism abundance, and co-metabolism status, and therefore, it is not applicable to perform biodegradation with a single species.

In the patent numbered CN104745502A in the state of the art, a microbacteria species and the use of this microbacteria in degrading organic pollutants are described. The strain described here was isolated from marine mud of the South China Sea and is a variant of mycobacterium. This variant (*Exiguobacterium profundum* L20, which can grow in extreme environments such as high salt) is inoculated into inorganic salt media containing 10-40% bisphenol A by volume. It is stated that the strain obtained in said invention can efficiently degrade environmental pollutants such as bisphenol A, but there is no information that it provides the degradation of other phenol compounds. In addition, since the strain is focused only on bisphenol A and cannot effectively degrade other phenol derivatives (e.g. phenol, catechol or other aromatic phenol derivatives), it is considered not to be sufficiently effective in the treatment of wastewater with complex phenol compound content (e.g. refinery wastewater). This situation limits the application area of this strain especially since refinery wastewater contains a wide variety of organic pollutants and requires high activity, and since it cannot degrade enough phenol compounds during the treatment of wastewater, it is not suitable for use in the treatment of refinery wastewater.

In the patent numbered CN104371948B in the state of the art, a microbacteria strain (a biologically pure culture of *Microbacterium sp.*) and the use of this strain in the degradation of phenol are described. Here, it is aimed to obtain phenol-degrading bacteria with high phenol tolerance and to purify phenol-containing wastewater with these bacteria. Said invention is stated as a biologically pure culture of the microbacteria species in a medium containing phenol as a carbon source and (NH₄)₂SO₄ as a nitrogen source. Although the invention mentions that phenol removal is provided in wastewater, it does not explain what these compounds are. In addition, refinery wastewater contains different phenol derivatives and various toxic organic compounds, and organisms dependent on a single carbon source cannot provide sufficient treatment against this diversity, therefore, they are not effective in the treatment of refinery wastewater. In addition, the use of microbacteria strains as pure cultures does not cause them to have a synergistic effect with natural microorganism consortia in the wastewater environment. While the use of consortia that can cooperate with microorganisms naturally found in the wastewater environment increases the treatment efficiency, pure cultures cause loss of efficiency in such complex systems. In addition, since the microbacteria strain in said invention is not obtained from wastewater, it will take a certain amount of time for the bacteria to adapt to the water they are placed in during the treatment process, and high concentrations of bacteria will be lost during this adaptation period.

The limitations and inadequacies of the solutions in the state of the art, the ineffectiveness of the treatment process due to the high cost of the methods used in the treatment of wastewater in the state of the art and the formation of secondary toxic substances, the inability of the microorganisms isolated from the wastewater system activated sludge to degrade phenol compounds at a sufficient level, the inadequacy of the adaptation levels of the microorganisms and their death at an early stage in the process and therefore their inability to perform their functions, and the inability of the microorganisms to degrade o-cresol, p-cresol, m-cresol, 2,3 dichlorophenol, 2,4 dichlorophenol, 3,4 dichlorophenol, 2,4 dinitrophenol and 2,5 dichlorophenol have made it necessary to develop a treatment for refinery wastewater.

### Brief Description and Aims of the Invention

In the invention, a bacterial culture mixture and the preparation method thereof to provide biodegradation of phenol compounds such as o-cresol, p-cresol, m-cresol, 2,3 dichlorophenol, 2,4 dichlorophenol, 3,4 dichlorophenol, 2,4 dinitrophenol, 2,5 dichlorophenol found in refinery wastewater is described. The bacterial mixture, which is the subject of the invention, comprises pure bacterial isolates isolated from wastewater system activated sludge belonging to the genera *Pseudomonas, Acinetobacter, Lysinibacillus, Bacillus, Kurthia, Comamonas, Microbacterium, Brevundimonas.* The bacterial culture mixture obtained by multiplying the isolates obtained from wastewater system activated sludge in the bioreactor, in the presence of the necessary growth medium and distilled water, provides treatment of refinery wastewater that exhibits high toxic activity for the ecosystem due to the presence of phenolic compounds.

The aim of the invention is to purify water by ensuring biodegradation of phenol compounds found in refinery wastewater. The bacterial isolates belonging to the genera *Pseudomonas, Acinetobacter, Lysinibacillus, Bacillus, Kurthia, Comamonas, Microbacterium, Brevundimonas,* which are the subject of the invention, provide the treatment of water by breaking down the phenol compounds o-cresol, p-cresol, m-cresol, 2,3 dichlorophenol, 2,4 dichlorophenol, 3,4 dichlorophenol, 2,4 dinitrophenol, 2,5 dichlorophenol in wastewater.

Another aim of the invention is to provide a bacterial culture mixture that quickly and easily adapts to the system to which they are given for the aim of treating refinery wastewater. The easy adaptation of said bacterial culture mixture to the system is possible by isolating the bacteria from the active sludge in refinery wastewater, which is the system in which they will be used. Since the bacteria currently found in refinery wastewater are adapted to the ambient conditions, the isolates used in the invention show rapid adaptation in the treatment process.

Another aim of the invention is to provide low-cost treatment of refinery wastewater. In the invention, the water treatment process is carried out at low cost by means of biological treatment. When biological treatment is compared to high-cost physical and chemical processing methods, it is accepted as one of the effective and economical ways to address phenol degradation through biodegradation. It is accepted that biological treatment is a promising method and an alternative to physicochemical techniques. Since the pollutants are decomposed enzymatically at ambient temperature, the method is less costly. In this way, phenol and other pollutants can be converted into inorganic mineral components at low cost.

Another aim of the invention is to provide a bacterial culture mixture that does not produce secondary toxic substances and does not exhibit antagonistic activity for use in the treatment of refinery wastewater. Said bacterial culture mixture does not produce secondary toxic substances due to the fact that they decompose by cometabolism and the intermediate products completely decompose into carbon dioxide (CO₂) and water. In addition, the bacteria in said bacterial mixture do not inhibit each other's degradation activities, so each species can show its activity at the maximum level.

### Description of Drawings

**Figure 1:** Biodegradation activity results

### Detailed Description of the Invention

The invention relates to a bacterial culture mixture and the preparation method thereof to provide biodegradation of phenol compounds such as o-cresol, p-cresol, m-cresol, 2,3 dichlorophenol (2,3 DCP), 2,4 dichlorophenol (2,4 DCP), 3,4 dichlorophenol (3,4 DCP), 2,4 dinitrophenol (2,4 DNP), 2,5 dichlorophenol (2,5 DNP) found in refinery wastewater. Said bacterial culture mixture comprises pure bacterial isolates isolated from wastewater system activated sludge belonging to the genera *Pseudomonas, Acinetobacter, Lysinibacillus, Bacillus, Kurthia, Comamonas, Microbacterium, Brevundimonas.* Said bacterial isolates are aerobically propagated in the presence of distilled water and growth media in the bioreactor, and the resulting mixture is used to degrade phenol compounds in refinery wastewater and treat water. Said growth media comprises 50-60% casein peptone, 5-10% dipotassium hydrogen phosphate, 5-10% glucose, 15-20% sodium chloride, and 5-10% soy peptone by weight.

Said bacterial culture mixture, which performs phenol elimination in order to provide biodegradation of phenol compounds, comprises 10-30% (v/v) by volume bacterial isolates belonging to the genera *Pseudomonas, Acinetobacter, Lysinibacillus, Bacillus, Kurthia, Comamonas, Microbacterium, Brevundimonas.* The bacteria in the mixture are present in equal proportions.

The bacteria belonging to the species in said bacterial mixture are isolated from the active sludge in the refinery wastewater system, thus said microorganisms adapt to the refinery wastewater environment. Therefore, during the use of these bacteria in the mixture for wastewater treatment, it takes a short time for the bacteria to adapt to the environment to which they are added, and their vitality levels are high. In addition, the bacteria in the invention do not form secondary toxic substances due to the fact that they degrade phenol compounds with co-metabolism (intermediate products are completely carbon dioxide (CO₂) and water) and do not inhibit the activity of one of them, in other words, they do not show antagonistic activity. Accordingly, the efficiency obtained from the degradation ability of each bacteria in the mixture is at the maximum level. Said bacterial mixture can be used in the treatment of petroleum refinery wastewater, especially in pharmaceutical industries, basic organic chemical manufacturing industries, petrochemical industries, coal gasification operations, gas liquefaction process, tannery, pesticide manufacturing industries and pulp and paper mill wastewater.

The method for preparing a bacterial culture mixture to provide biodegradation of the phenol compounds of the invention comprises the process steps of;
i. growing bacteria of the genera *Pseudomonas, Acinetobacter, Lysinibacillus, Bacillus, Kurthia, Comamonas, Microbacterium, Brevundimonas* in a standard microbial agar growth medium to which approximately 1-10 ppm phenol has been added in an oven at 25 - 30°C for 12-48 hours to allow acclimation,
ii. adjusting optical density (OD) of bacterial isolates belonging to the genera *Pseudomonas, Acinetobacter, Lysinibacillus, Bacillus, Kurthia, Comamonas, Microbacterium, Brevundimonas* by diluting them in physiological saline after incubation, and
iii. obtaining a bacterial culture mixture comprising bacterial isolates of the genera *Pseudomonas, Acinetobacter, Lysinibacillus, Bacillus, Kurthia, Comamonas, Microbacterium, Brevundimonas* at a ratio of 10-30% (v/v) by volume by adding 5-10% (v/v) by volume to the growth medium from the bacteria whose ODs have been adjusted and growing them at 25-30°C, 120-300 rpm for 12-48 hours.

In the preparation method of the bacterial mixture which is the subject of the invention, inoculation is made at a volume of 5-10% by volume (approximately 10-10¹⁰ cfu/mL for each bacterial isolate) into a growth medium at a volume of 0.05-5% by weight and distilled water at a volume of 70-95% by volume in a bioreactor (Sartorius Stedim Biostat^{®} A) with a volume of 5 litres; aerobic reproduction of the bacteria is carried out for approximately 12-48 hours according to the growth curve results at 25°C-30°C, 6-9 pH, 120 - 300 rpm. Each isolate inoculated into the bioreactor and whose optical density has been adjusted is cultivated with the pour plate method during production.

The final mixture comprises bacteria belonging to the genera *Pseudomonas, Acinetobacter, Lysinibacillus, Bacillus, Kurthia, Comamonas, Microbacterium, Brevundimonas* at a rate of 10-30% by volume. Each bacterial species is present at the same rate in the mixture containing 10-30% by volume bacteria. Said bacterial culture mixture is added to water containing real wastewater for the aim of treating wastewater. In the invention, phenols and phenolic compounds in the water environment are degraded by microorganisms as the only carbon and energy source. A significant disadvantage of all these cultures in the literature is that they have narrow specificity for a certain substrate and can degrade a limited number of phenolic compounds. Most of the effective phenol reducing microorganisms have the ability to use phenol as the sole carbon and energy source for their growth and metabolism. Said bacterial mixture first converts phenol to catechol with the help of hydroxylase enzyme. Catechol is then reduced to its intermediate products by ortho or meta cleavage with the help of enzymes. Our said bacterial mixture is degraded by the bacterial mixture with the help of various enzymes through the tricarboxylic acid cycle (TCA) resulting in CO₂, metabolites and energy via meta pathway. The final bacterial isolates are in the aqueous mixture with a growth media containing distilled water and 50-60% by weight casein peptone, 5-10% dipotassium hydrogen phosphate, 5-10% glucose, 15-20% sodium chloride, 5-10% soy peptone.

The biodegradation activity results of the prepared product are given in Figure 1. The final mixture comprises bacteria belonging to the genera *Pseudomonas, Acinetobacter, Lysinibacillus, Bacillus, Kurthia, Comamonas, Microbacterium, Brevundimonas* at a rate of 10-30% by volume. In the graph of said mixture (Figure 1), a concentration is selected and the time period showing the end of the chemicals (from 0 to 48 hours) is shown. Different experiments were conducted in the chemical concentration range of 50-100 mg/L (ppm) (including all target chemicals) and it was observed that the mixture degraded the targeted chemicals at different times within 0 - 48 hours.

### REFERENCES

**[1]**Institute, H., & The Hydraulic Institute (HI) is the global authority on pumps and pumping systems. HI is a pump association of positive displacement and rotodynamic. (2021, January 4). What is the definition of wastewater? Pumps and Systems Magazine. Retrieved December 15, 2022.
**[2]**Wastewater. Wastewater - an overview | ScienceDirect Topics. (n.d.). Retrieved December 15, 2022.
**[3]**Canada, E. and C. C. (2014, April 11). *Government of Canada.* Canada.ca. Retrieved December 15, 2022.
**[4]**Nemerov, N.L. (Ed.). (2009). Environmental Engineering - Water, Wastewater, Soil and Groundwater Treatment and Remediation. (6th edition). New Jersey: John Wiley & Sons, Inc.
**[5]**De Zuane, J. (1990). Handbook of Drinking Water Quality. (2nd edition). ABD: John Wiley & Sons Inc.
**[6]**Anku, W. W., Mamo, M. A., & Govender, P. P. (2017, March 15). Phenolic compounds in water: Sources, reactivity, toxicity and treatment methods. IntechOpen. Retrieved December 15, 2022.
**[7]**Maguire, P. (2022, November 17). Removing highly toxic phenolic compounds: Wastewater treatment options. Saltworks Technologies. Retrieved December 15, 2022.
**[8]**Kulkarni, S., & Kaware, D. (2013). Review on Research for Removal of Phenol from. International Journal of Scientific and Research Publications
**[9]**Quality and function of anaerobic digestion residues. SLUpub. (n.d.). Retrieved December 16, 2022.
**[10]**Hinteregger C;Leitner R;Loidl M;Ferschl A;Streichsbier F; (n.d.). Degradation of phenol and phenolic compounds by pseudomonas putida EKII. Applied microbiology and biotechnology. Retrieved December 16, 2022.

## Claims

1. A bacterial culture mixture for biodegradation of phenol compounds in refinery wastewater, comprising bacterial isolates belonging to the genera *Pseudomonas, Acinetobacter, Lysinibacillus, Bacillus, Kurthia, Comamonas, Microbacterium* and *Brevundimonas* at 10-30% (v/v) by volume.

2. A bacterial culture mixture according to Claim 1, wherein the phenol compounds that are biodegraded are o-cresol, p-cresol, m-cresol, 2,3 dichlorophenol, 2,4 dichlorophenol, 3,4 dichlorophenol, 2,4 dinitrophenol or 2,5 dichlorophenol.

3. A mixture of bacterial cultures according to Claim 1 or Claim 2, wherein said bacterial isolates are present in equal proportions.

4. The preparation method of bacterial culture mixture to provide biodegradation of phenol compounds, comprising the process steps of:
i. growing bacteria of the genera Pseudomonas, Acinetobacter, Lysinibacillus, Bacillus, Kurthia, Comamonas, Microbacterium, Brevundimonas in a standard microbial agar growth medium to which approximately 1-10 ppm phenol has been added in an oven at 25 - 30°C for 12-48 hours to allow acclimation,
ii. adjusting optical density (OD) of bacterial isolates belonging to the genera Pseudomonas, Acinetobacter, Lysinibacillus, Bacillus, Kurthia, Comamonas, Microbacterium, Brevundimonas by diluting them in physiological saline after incubation, and
iii. obtaining a bacterial culture mixture comprising bacterial isolates of the genera *Pseudomonas, Acinetobacter, Lysinibacillus, Bacillus, Kurthia, Comamonas, Microbacterium, Brevundimonas* at a ratio of 10-30% (v/v) by volume by adding 5-10% (v/v) by volume to the growth medium from the bacteria whose ODs have been adjusted and growing them at 25-30°C, 120-300 rpm for 12-48 hours.

5. A bacterial culture mixture prepared by a method according to claim 4.
